# EUROPEAN PATENT SPECIFICATION

(11) **EP 1 701 698 B1**
(45) Date of publication and mention of the grant of the patent: **16.01.2008**
(21) Application number: 04814371.3
(22) Date of filing: 14.12.2004
(51) Int. Cl.: A61K 31/4745, A61K 9/20, A61K 9/48

(54) **DIRECTLY COMPRESSIBLE PHARMACEUTICAL COMPOSITION FOR THE ORAL ADMIMISTRATION OF CCI-779**
DIREKT KOMPRIMIERBARE PHARMAZEUTISCHE ZUSAMMENSETZUNG FÜR DIE ORALE VERABREICHUNG VON CCI-779
COMPOSITION PHARMACEUTIQUE DIRECTEMENT COMPRESSIBLE POUR L'ADMINISTRATION ORALE DE CCI-779

(30) Priority: 08.01.2004 US 534951 P
(43) Date of publication of application: 20.09.2006
(73) Proprietor: Wyeth, Madison, NJ 07940 (US)
(72) Inventor: ASHRAF, Muhammad, Elmwood Park, NJ 07407 (US); BENJAMIN, Eric, J., Jamestown, NC 27282 (US)
(74) Representative: Wileman, David Francis
(86) International application number: PCT/US2004/042178
(87) International publication number: WO 2005/070393

(56) References cited:
- US-A- 5 362 718
- US-A1- 2002 091 137

## Description

### BACKGROUND OF THE INVENTION

Rapamycin 42-ester with 3-hydroxy-2-(hydroxymethyl)-2-methylpropionic acid (CCI-779) is an anticancer agent and is characterized by the following structure.

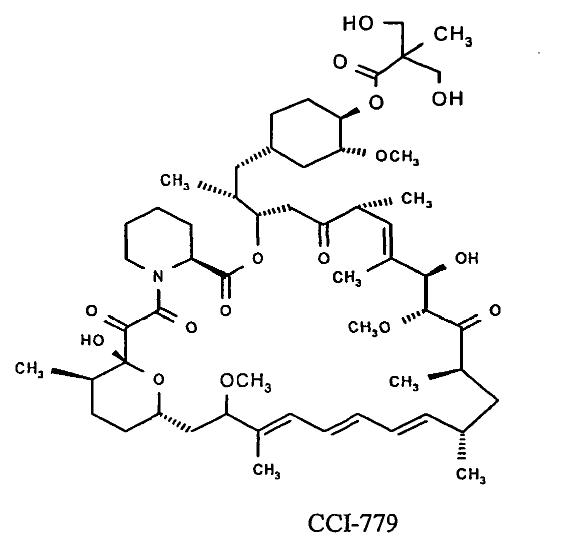

CCI-779 exhibits cytostatic, as opposed to cytotoxic properties, and may delay the progression of tumors or tumor recurrence. The mechanism of action of CCI-779 that results in the G1 to S phase block is novel for an anticancer drug. *In vitro,* CCI-779 has been shown to inhibit the growth of a number of histologically diverse tumor cells. Central nervous system (CNS) cancer, leukemia (T-cell), breast cancer, prostate cancer, and melanoma lines were among the most sensitive to CCI-779. The compound arrested cells in the G1 phase of the cell cycle.

CCI-779 has poor water solubility (less than 1 µg/ml) and high permeability (Log PC ≥ 4.1 in 1-octanol/water system and P_{eff} = 4-5 X 10⁻⁵ cm/sec obtained from *in situ* rat intestinal perfusion study using metoprolol tartrate as a marker) and is classified as class II compound according to BCS classification system. One obstacle towards the formulation of CCI-779 is its poor aqueous dissolution and low oral bioavailability:
Additionally, CCI-779 exhibits aqueous instability and has shown its potential to undergo oxidation.

US 5362718 discloses powder and tablet formulations comprising a carrier and a rapamycin ester like e.g CCI-779 in admixture.

A CCI-779 formulation was developed that employed a wet granulation manufacturing process. US Published Patent Application, Publication No. US- 2004-0077677-A1. This process involved preparation of a hydroalcoholic granulation solution of CCI-779. Further, although the resulting tablets were stable and bioavailable, the preparation of the hydroalcoholic solution was very tedious. Further, CCI-779 was thermodynamically unstable, precipitating within one day after its preparation, requiring it to be used immediately after its preparation.

In view of this, a simple manufacturing process is required that can produce stable and bioavailable tablets and can be used for commercial manufacturing.

### SUMMARY OF THE INVENTION

The present invention provides a convenient and effective method to deliver therapeutic levels of CCI-779 to the patient. The invention provides pharmaceutical compositions containing a stable and bioavailable form of micronized CCI-779, and optionally, an antioxidant or a chelating agent, or mixtures thereof, in an immediate release dosage form for oral administration. The composition is in the form of a tablet or in filled capsules.

Other aspects and advantages of the invention will be readily apparent from the following detailed description of the invention.

### DETAILED DESCRIPTION OF THE INVENTION

The invention provides micronized CCI-779, that can be readily formulated into an oral dosage unit, and is particularly well suited for a directly compressible unit. The inventors have found that tablets prepared by direct compression of micronized CCI-779 formulations of the invention exhibited rapid and complete drug release, as compared to nonmicronized CCI-779, even when the nonmicronized CCI-779 was formulated with surfactants. See, *e.g.,* Example 4. Thus, the compositions of the invention provide fast drug release.

Briefly, CCI-779 is micronized under nitrogen and conventional micronizing techniques, for example with a Trost or jet mill, applied to non-micronized CCI-779. The preparation of non-micronized CCI-779 is described in U.S. Patent 5,362,718, which is hereby incorporated by reference. A regioselective preparation of non-micronized CCI-779 is described in US Patent 6,277,983, which is hereby incorporated by reference. However, the invention is not limited to the method by which the non-micronized CCI-779 is produced. Micronized CCI-779 typically has a particle size of about 0.2 to about 30 microns, about 0.5 to 25 microns, or about 0.5 to 20 microns, as described above.

The compositions of the invention contain micronized CCI-779 with a particle size range of less than or equal to about 3 microns (µm), 50% are about 10 µm, and 90% are less than or equal to about 20 µm as determined by Malvern method. In one embodiment, the micronized CCI has a particle size range of 10% are less than or equal to about 2 µm, 50% are about 5 µm, and 90% are less than or equal to about 16 µm as determined by Malvern method.

Suitably, the micronized CCI-779 is present in the composition of the invention in an amount from 0.1 % w/w to 50% w/w, based on the weight of an uncoated composition of the invention. This amount may be varied, depending upon the amount of micronized CCI-779 to be delivered to a patient. For example, an effective amount of micronized CCI-779 is generally in the range, *e.g.,* about 0.1 to about 50 mg, about 10 mg to about 30 mg, or about 0.5 to about 2 mg micronized CCI-779. The desired therapeutic regimen can be taken into consideration when formulating a composition of the invention. For example, micronized CCI-779 can be in the range of 0.1% w/w to 10% w/w for an uncoated composition of the invention. In another example, micronized CCI-779 can be in the range of 5% w/w to 25% w/w based upon the weight of an uncoated unit dose. In yet another example, micronized CCI-779 can be in the range of 6% w/w to 8% w/w, 15% w/w to 40% w/w, or 20% w/w to 30% w/w based on the weight of an uncoated unit dose.

In addition to containing micronized CCI-779, the composition of the present invention can contain pharmaceutically acceptable additives and/or excipients. Typically, these additives are biologically inert and useful for manufacture of a dosing unit. The compositions of the invention may contain one or more filler/binder, disintegrant, a dissolution enhancer (including, e.g., a surfactant), glidant, and lubricant. In certain embodiments, the compositions further contain one or more antioxidants, chelating agents, or pH modifiers. Optionally, the antioxidant, chelating agent, and/or pH modifier may be micronized. Micronized additives and excipients are prepared using conventional techniques, as described.

Examples of pharmaceutically acceptable binders, fillers, and disintegrants include sucrose, lactose, magnesium stearate, gum acacia, cholesterol, tragacanth, stearic acid, gelatin, casein, lecithin (phosphatides), carboxymethylcellulose calcium, carboxymethylcellulose sodium, methylcellulose, hydroxyethylcellulose, hydroxypropylcellulose, hydroxypropylmethycellulose phthalate, noncrystalline cellulose, cetostearyl alcohol, cetyl alcohol, cetyl esters wax, dextrates, dextrin, lactose, dextrose, glyceryl monooleate, glyceryl monostearate, glyceryl palmitostearate, polyoxyethylene alkyl ethers, polyethylene glycols, polyoxyethylene castor oil derivatives, polyoxyethylene stearates, and polyvinyl alcohol, and the like.

In one embodiment, the disintegrant is croscarmellose sodium. Suitably, a composition of the invention contains a total of about 3% w/w to 8% w/w disintegrant, e.g., about 4% to about 6% w/w.

In one embodiment, the binders and fillers are selected from the group consisting of polyvinylpyrrolidone (povidone), lactose (including anhydrous lactose), and microcrystalline cellulose, and mixtures thereof. Suitably, a composition of the invention contains a total of about 75 % w/w to 88% w/w binder/filler, or about 80% w/w to 82% w/w binder/filler, based on the weight of an uncoated composition. For example, a composition of the invention may contain, in addition to the micronized CCI-779 and other components, about a low amount of povidone, e.g. about 5 to 7% w/w, and more desirably, about 6% w/w, with the remainder of the filler in the uncoated composition being supplied by other components. In another example, a composition of the invention may contain a high amount of povidone, e.g., about 25 to 35% w/w, and more desirably, about 30 to 32 % w/w povidone, with the remainder of the filler in the uncoated composition being supplied by other components. In yet another example, a composition of the invention contains a combination of lactose, preferably anhydrous lactose, and microcrystalline cellulose, optionally with povidone or another filler/binder. In such a composition (based on uncoated weight), anhydrous lactose is generally present in an amount of about 30% w/w to about 60% w/w, and more desirably, about 30 % w/w, about 32% w/w, about 50% w/w, or about 55% w/w anhydrous lactose. Suitably, in such an uncoated composition, microcrystalline cellulose is present in an amount of about 15% w/w to about 30% w/w of the uncoated composition, and more desirably, about 16% w/w, about 23% w/w, about 25% w/w, about 28% w/w of the uncoated composition.

Dissolution enhancers may be included in the micronized CCI-779 composition (based on uncoated weight) of the invention. Preferably, one or more dissolution enhancers may optionally be present in the composition in an amount of from about 0.5 % w/w to about 10 % w/w, and preferably, from about 5% w/w to about 8% w/w, about 5.5%, about 6% w/w, or 6.5% w/w, based on the weight of an uncoated composition. Examples of dissolution enhancers include surfactants, chelating agents (*e.g*., EDTA), disintegrants, or combinations thereof.

In one embodiment, the surfactant is about 0.25 % w/w to about 10 % w/w of an uncoated composition, and preferably, about 5% w/w to about 6.5% w/w. In one embodiment, the surfactant is selected from sodium lauryl sulfate (also known as sodium dodecyl sulfate). Other suitable surfactants are well known to those of skill in the art and can be selected from, without limitation, polysorbates including, e.g., polysorbate 80, Polaxamer 188™ surfactant, sodium lauryl sulfate (sodium dodecyl sulfate), salts of bile acids (taurocholate, glycocholate, cholate, deoxycholate, etc. ) which may be combined with lecithin. Alternatively, ethoxylated vegetable oils, such as Cremophor EL, vitamin E tocopherol propylene glycol succinate (Vitamin E TGPS), polyoxyethylene-polyoxypropylene block copolymers, and poloxamers may be selected.

Acceptable antioxidants include, but are not limited to, citric acid, d,1-α-tocopherol, butylated hydroxyanisol (BHA), butylated hydroxytoluene (BHT), monothioglycerol, ascorbic acid, propyl gallate, and mixtures thereof. It is expected that the total amount of antioxidants in the formulations of this invention will be in concentrations ranging from 0.001 % to 3% w/w, and preferably, about 0.01 w/w to about 1% w/w, and more preferably, about 0.02 % w/w to 0.1 % w/w, based on the weight of an uncoated composition. In one embodiment, the antioxidant is a combination of BHA and BHT, which may be in nonmicronized form or preferably, in micronized form.

Chelating agents and other materials capable of binding metal ions, such as ethylene diamine tetra acetic acid (EDTA) and its salts and hydrates (e.g., EDTA calcium disodium hydrous) are useful in the compositions of the invention. Typically, where present, a chelating agent is present in an amount less than 1 % w/w, e.g., about 0.001 % w/w to about 0.01 % w/w, based on the weight of an uncoated composition. In one embodiment, the chelating agent is present in micronized form.

Acceptable pH modifying agents include, but are not limited to citric acid and salts thereof (e.g., sodium citrate), dilute HCl, and other mild acids or bases capable of buffering a solution containing CCI-779 to a pH of 4 to 6. Where present in a composition of the invention, such pH modifiers are present in an amount up to about 1% w/w, e.g., about 0.001% w/w to about 0.1% w/w, based on the weight of an uncoated composition. Optionally, the pH modifier, can be present in micronized form.

Other suitable components include lubricants and/or glidants. In one embodiment, the lubricant and the glidants can each be present in the composition of the invention in an amount of 0.01 wt% to about 1 wt%, about 0.1 wt% to about 2 wt%, or about 0.2 to about 0.5%, of an uncoated composition. In some embodiments, the lubricant and glidants are present in the composition in amounts of less than 1 wt% of an uncoated composition. An example of a suitable lubricant is magnesium stearate and an example of a suitable glidants is silicone dioxide.

Other suitable inert components of the formulation will be readily apparent to one of skill in the art.

The compositions of the invention are formed into a suitable dosing unit for delivery to a patient. Suitable dosing units include oral dosing units, such as a directly compressible tablet, a capsule, a powder and a suspension. The compositions of the invention can also be formulated for delivery by other suitable routes. These dosing units are readily prepared using the methods described herein and those known to those of skill in the art.

In one embodiment, a composition of the invention is prepared by dry mixing micronized CCI-779 with the other additives in a suitable mixer. The powder mix is then directly compressed into unit dose tablets.

Without limitation as to the method of preparation of a composition of the invention, an example of a suitable micronized CCI-779 formulation includes a low amount of povidone. The following weight percentages are based upon an uncoated composition of the invention.

| | |
|---|---|
| CCI-779, Micronized | 6% w/w; |
| Sodium Lauryl Sulfate | 6% w/w; |
| Povidone | 6% w/w; |
| Lactose Anhydrous | 50 % w/w; |
| Microcrystalline Cellulose | 25% w/w; |
| Croscarmellose Sodium | 6% w/w; |
| Glidant | 0.25% w/w; and |
| Magnesium Stearate | 0.25% w/w. |

Still a further example of a suitable micronized CCI-779 composition contains a high amount of povidone, with weight percentages based upon an uncoated composition of the invention:

| | |
|---|---|
| Micronized CCI-779 | 6 % w/w; |
| Sodium Lauryl Sulfate | 6% w/w; |
| Povidone | 31 % w/w; |
| Lactose Anhydrous | 34% w/w; |
| Microcrystalline Cellulose | 16% w/w; |
| Croscarmellose Sodium | 6% w/w ; |
| Glidant | 0.25% w/w; and |
| Magnesium Stearate | 0.5% w/w. |

Yet a further example of a suitable micronized CCI-779 dosing unit, with weight percentages based on total uncoated composition, is:

| | |
|---|---|
| Micronized CCI-779 | 6% w/w; |
| Butylated Hydroxyanisol | 0.022% w/w; |
| Butylated Hydroxytoluene | 0.05% w/w; |
| EDTA | 0.011% w/w; |
| Citric acid | 0.08% w/w |
| Poloxamer 188 | 6% w/w |
| Lactose Anhydrous | 55% w/w |
| Microcrystalline Cellulose | 28 w/w |
| Croscarmellose Sodium | 4% w/w |
| Glidant | 0.25% w/w; and |
| Magnesium Stearate | 0.5% w/w. |

Yet another example of a suitable dosing unit, with weight percentages based on total uncoated composition, is:

| | |
|---|---|
| CCI-779 (Micronized) | 6% w/w; |
| Butylated Hydroxyanisole (Micronized) | 0.022% w/w; |
| Butylated Hydroxytoluene (Micronized) | 0.05% w/w; |
| EDTA Calcium Disodium, Hydrous (Micronized) | 0.011% w/w; |
| Citric Acid Anhydrous (Micronized) | 1 % w/w; |
| Sodium Lauryl Sulfate | 6% w/w; |
| Povidone K-25 | 65% w/w; |
| Microcrystalline Cellulose | 23% w/w; |
| Anhydrous Lactose | 50% w/w; |
| Croscarmellose Sodium | 6 % w/w; |
| Colloidal Silicone Dioxide | 0.25% w/w; and |
| Magnesium Stearate | 0.50% w/w. |

Optionally, the tablets are film-coated. Suitable film-coatings are known to those of skill in the art. For example, the film-coating can be selected from among suitable polymers such as hydroxypropylmethylcellulose, ethyl cellulose, polyvinyl alcohol, and combinations thereof. Such coatings may also contain placticizers and other desirable components. In one embodiment, the coatings are inert. Other suitable film-coatings can be readily selected by one of skill in the art. Where applied, the weight percent of the film coat is generally in the range of 1 % w/w to 6 % w/w, about 2 % w/w, about 3% w/w, about 4% w/w or about 5% w/w, and more desirably, about 2% w/w, based on the total weight of the coated composition.

The invention further provides a method of delivering CCI-779 to a patient, said method comprising the step of administering a micronized CCI-779 dosing unit according to the invention.

It is contemplated that when the formulations of this invention are used as an immunosuppressive or anti-inflammatory agent, they can be administered in conjunction with one or more other immunoregulatory agents. Such other antirejection chemotherapeutic agents include, but are not limited to azathioprine, corticosteroids, such as prednisone and methylprednisolone, cyclophosphamide, cyclosporin A, FK-506, OKT-3, and ATG. By combining one or more of the formulations of the present invention with such other drugs or agents for inducing immunosuppression or treating inflammatory conditions, lesser amounts of each of the agents may be required to achieve the desired effect. See, e.g., *Transplantation Proc.* **23:** 507 (1991).

The dosage requirements may vary the severity of the symptoms presented and the particular subject being treated. Daily oral dosages of micronized CCI-779 can be 0.05 to 30 mg, about 1 mg to 25 mg, about 5 mg to about 10 mg. In one example, micronized CCI-779 is used in combination therapy at daily doses in the range of 0.5 to 10 mg. In another example, micronized CCI-779 is used in monotherapy at daily doses in the range of 1 mg to 30 mg. In other embodiments, daily doses are 2 to 5 mg when micronized CCI-779 is used in combination therapy, and 5 to 15 mg when micronized CCI-779 is used as monotherapy.

Treatment can be initiated with small dosages less than the optimum dose of the compound. Thereafter the dosage is increased until the optimum effect under the circumstances is reached. Precise dosages will be determined by the administering physician based on experience with the individual subject treated. In general, the formulations of this invention are most desirably administered at a concentration that will generally afford effective results without causing any unacceptable harmful or deleterious side effects.

### EXAMPLE 1: Directly Compressible Tablet Formulations Prepared By Employing Non-Micronized CCI-779

The compositions included in this example employed non-micronized CCI-779 and were prepared with or without a surfactant. The tabletting was carried out by dry blending and direct compression method.

**Table 1: Quantitative Composition of CCI-779 Tablets, 5mg Containing Non-Micronized CCI-779 without Surfactant**

| Ingredients | Percent Wt/Wt | Mg/tablet | Function |
|---|---|---|---|
| CCI-779, non-micronized | 1.44 | 5.00 | Active |
| Butylated Hydroxyanisol, NF | 0.1 | 0.35 | Antioxidant |
| Butylated Hydroxytoluene, NF | 0.05 | 0.18 | Antioxidant |
| EDTA, USP | 0.01 | 0.04 | Chelating agent |
| Sodium Citrate Anhydrous | 0.75 | 2.62 | pH modifier |
| Citric acid, Anhydrous USP | 0.25 | 0.87 | pH modifier |
| Povidone, K17, USP | 7.14 | 24.99 | Filler/ Binder |
| Lactose Anhydrous, NF | 34.30 | 120.05 | Filler |
| Microcrystalline Cellulose,NF (Avicel PH 112 ) | 51.46 | 180.11 | Filler/ Binder |
| Croscarmellose Sodium, NF | 4.00 | 14.0 | Disintegrant |
| Magnesium Stearate, NF | 0.50 | 1.75 | Lubricant |
| Total | 0.05 | 350 | |

**Table 2: Quantitative Composition of CCI-779 Tablets, 25 mg Containing Non-Micronized CCI-779 and Surfactant**

| Ingredients | Percent wt/Wt | mg/tablet | Function |
|---|---|---|---|
| CCI-779, non-micronized | 6.25 | 25.00 | Active |
| Sodium Lauryl Sulfate, NF | 5.625 | 22.50 | Surfactant |
| Povidone, K17, USP | 31.25 | 125.00 | Filler/ Binder |
| Lactose Anhydrous, NF | 33.75 | 135.00 | Filler |
| Microcrystalline Cellulose,NF (Avicel PH 112) | 16.375 | 65.50 | Filler/ Binder |
| Croscarmellose Sodium, NF | 6.0 | 24.00 | Disintegrant |
| Silicone dioxide (Aerosil 200) | 0.25 | 1.00 | Glidant |
| Magnesium Stearate, NF | 0.50 | 2.00 | Lubricant |
| Total | 100 | 400 | |

CCI-779 tablets prepared by direct compression of nonmicronized CCI-779 with standard excipients and fillers, in the presence or absence of surfactants yielded tablets that did not exhibit rapid and complete drug release, and thereby resulted in an unsuitable formulation for CCI-779.

### EXAMPLE 2: Directly Compressible Tablet Formulations Prepared By Employing Micronized CCI-779, Sodium Lauryl Sulphate And Povidone

The tablet formulations for this example are manufactured using the following protocol.

Microcrystalline cellulose (Avicel PH-112) and povidone K-25 are passed through a screen and transferred to a V-blender of suitable size. Micronized CCI-779 is preblended with a portion of lactose anhydrous separately, then passed through a screen and added to the V-blender. Sodium lauryl sulfate, croscarmellose sodium, silicone dioxide and a portion of lactose anhydrous are passed through a screen and transferred to the V blender. The remaining lactose anhydrous is passed through a screen and transferred it to V-blender and the lids are closed. The material is blended without activation of intensifier bar. Magnesium stearate is passed through a screen, premixed with a weight equivalent portion of powder, blended from V-blender, transferred to the lubricant premix to V-blender and blended without activation of intensifier bar. The final blend is compressed using a tablet press with suitable tooling.

**Table 3: Quantitative Composition of CCI-779 Tablets, 25 mg Containing Low level of Povidone**

| **Ingredients** | **Percent Wt/Wt** | **Mg** / **tablet** | **Function** |
|---|---|---|---|
| CCI-779, Micronized | 6.250 | 25.00 | Active |
| Sodium Lauryl Sulfate, NF | 5.625 | 22.50 | Surfactant |
| Povidone, USP K25 | 6.250 | 25.00 | Filler/ Binder |
| Lactose Anhydrous, NF | 50.583 | 202.33 | Filler |
| Microcrystalline Cellulose,NF ( Avicel PH 112 ) | 24.543 | 98.172 | Filler/ Binder |
| Croscarmellose Sodium, NF | 6.000 | 24.00 | Disintegrant |
| Aerosil 200, NF | 0.250 | 1.00 | Glidant |
| Magnesium Stearate, NF | 0.500 | 2.00 | Lubricant |
| Total | 100 | 400 | |

**Table 4: Quantitative Composition of CCI-779 Tablets, 25 mg Containing High Level of Povidone**

| Ingredients: | Percent Wt/Wt | Mg / tablet | Function |
|---|---|---|---|
| CCI-779, Micronized | 6.250 | 25.00 | Active |
| Sodium Lauryl Sulfate, NF | 5.625 | 22.50 | Surfactant |
| Povidone, USP K-25 | 31.250 | 125.00 | Filler/ Binder |
| Lactose Anhydrous, NF | 33.750 | 135.00 | Filler |
| Microcrystalline Cellulose, NF | 16.375 | 65.50 | Filler/ Binder |
| (Avicel PH 112) | | | |
| Croscarmellose Sodium, NF | 6.000 | 24.00 | Disintegrant |
| Silicone dioxide (Aerosil 200), NF | 0.250 | 1.00 | Glidant |
| Magnesium Stearate, NF | 0.500 | 2.00 | Lubricant |
| Total | 100 | 400 | |

### EXAMPLE 3: Directly Compressible Tablet Formulations Prepared By Employing Micronized CCI-779 And Poloxamer As Surfactant

The table formulations for this example are manufactured according to the following protocol.

Pass the poloxamer 188, microcrystalline cellulose (Avicel PH-112) and a portion of anhydrous lactose through a screen and blend. Mill the blend containing poloxamer with the help of a Fitz mill and transfer it to a V-blender of suitable size.

Preblend a portion of anhydrous lactose with micronized butylated hydroxyanisole, butylated hydroxytoluene, EDTA calcium disodium, hydrous, and citric acid anhydrous. Then add-CCI-779 to this preblend, mix and add to the V-blender.

Take a portion of anhydrous lactose, croscarmellose sodium, and colloidal silicon dioxide (Aerosil 200) and pass through a screen, blend and transfer it to V-blender. Pass the remaining anhydrous lactose through a screen and transfer it to V-blender. Close the lids and blend the material without activation of the intensifier bar. Pass magnesium stearate through a screen, premix with a weight equivalent portion of powder blend and transfer the lubricant premix to V-blender and blend without the activation of the intensifier bar. Compress the final blend using a tablet press equipped with suitable tooling.

**Table 5: Quantitative Composition of CCI-779 Tablets, 25 mg Containing Poloxamer**

| **Ingredients:** | **Percent Wt/Wt** | **Mg / tablet** | **Function** |
|---|---|---|---|
| CCI-779, Micronized | 6.250 | 25.00 | Active |
| Butylated Hydroxyanisol, NF | 0.022 | 0.088 | Antioxidant |
| Butylated Hydroxytoluene, NF | 0.050 | 0.20 | Antioxidant |
| EDTA, calcium disodium | 0.011 | 0.044 | Chelating agent |
| hydrous, USP | | | |
| Citric acid, Anhydrous USP | 0.080 | 0.32 | pH modifier |
| Poloxamer 188, NF | 6.250 | 25.00 | Surfactant |
| Lactose Anhydrous, NF | 55.060 | 220.24 | Filler |
| Microcrystalline Cellulose, NF | 27.527 | 108.58 | Filler/ Binder |
| (Avicel PH 112) | | | |
| Croscarmellose Sodium, NF | 4.000 | 16.00 | Disintegrant |
| Aerosil 200, NF | 0.250 | 1.00 | Glidant |
| Magnesium Stearate, NF | 0.500 | 2.00 | Lubricant |
| **Total** | 100 | 400 | |

### EXAMPLE 4 - Dissolution of CCI-779 Tablets

All the CCI-779 tablet formulations were evaluated by dissolution test. Dissolution test was performed using USP method II in 500 ml of 0.4% sodium lauryl sulfate at 75 RPM paddle speed. Table 6 summarizes the dissolution characteristics of the neat CCI-779 API and various tablet formulations of CCI-779.

**Table 6: Dissolution Data of CCI-779 Tablet Formulations**

| | **Percent CCI-779 Dissolved** | | | | | |
|---|---|---|---|---|---|---|
| **Time (min)** | **CCI-779 (API) L21296-119*** | **Batch Tablet 1** | **Batch Table 2** | **Batch Table 4** | **Batch Table 3** | **Batch Table 5** |
| 10 | 4 | 31 | 30 | 56 | 87 | 90 |
| 20 | 9 | 42 | 58 | 87 | 96 | 94 |
| 30 | 14 | 50 | 74 | 95 | 98 | 95 |
| 45 | 21 | 56 | 86 | 97 | 99 | 97 |
| 60 | ---- | ---- | 93 | 98 | 100 | 97 |

| | | | | | | |
|---|---|---|---|---|---|---|
| * Pure CCI-779 was filled in hard gelatin capsules to test its dissolution. | | | | | | |

Dissolution results in Table 6 show that the tablets prepared by direct compression method (Table 1) did not exhibit rapid and complete drug release. Even the addition of a surfactant (Table 2) did not enhance dissolution of CCI-779 from these tablets. However, the directly compressible compositions of the invention containing micronized CCI-779 (Tables 3, 4, 5) exhibited fast and complete drug release.

### EXAMPLE 5: Bioavailability of CCI-779 in Human - Evaluation of Oral Dosage Forms

The three prototype tablet formulations containing micronized CCI-779 (Tables 3, 4 and 5) were further evaluated for absorption in human volunteers. A previously used clinical formulation prepared by wet granulation process was used as a control. The results of this biostudy are shown in Table 7 below.

The following chart relates the treatments of the bio-study to the compositions in this document and respective batch numbers of clinical batches:

| **Treatment** | **Composition in** |
|---|---|
| High Povidone | Table 3 |
| Low Povidone | Table 4 |
| Poloxamer | Table 5 |
| Control | -- |

**Table 7: Pharmacokinetic Parameters (± S.D) Following Oral Administration of GCI-779 Tablet, 25 mg to Human Volunteers**

| **Treatment** | **t _{½} (hr)** | **Cₘₐₓ (ng/ml)** | **Tₘₐₓ (hr)** | **AUC _{0-∞} (ng.hr/ml)** |
|---|---|---|---|---|
| Low | 79.5 | 17.06 | 2.16 | 554.6 |
| Povidone | (17.0) | (8.07) | (0.9) | (187.7) |
| High | 81.8 | 18.7 | 2.86 | 575.4 |
| Povidone | (23.7) | (9.6) | (2.1) | (190) |
| Poloxamer | 77.9 | 11.36 | 4.08 | 544.0 |
| | (18.7) | (7.0) | (2.12) | (150.4) |
| | 81.8 | 27.458 | 1.39 | 664.1 |
| Control | (17.2) | (12.4) | (0.637) | (217.5) |

### EXAMPLE 6: CCI-779 10 mg Film Coated Tablet Formulation Prepared By Employing Micronized CCI-779

**Table 8: Quantitative Composition of CCI-779 Tablets, 10 mg**

| **Ingredients:** | **Percent Wt/Wt** | **mg** / **tablet** | **Function** |
|---|---|---|---|
| CCI-779, Micronized | 6.25 | 10.00 | Active |
| Butylated Hydroxyanisol, Micronized | 0.022 | 0.035 | Antioxidant |
| Butylated Hydroxytoluene, Micronized | 0.050 | 0.080 | Antioxidant |
| EDTA, Calcium Disodium, Hydrous, Micronized | 0.011 | 0.044 | Chelating agent |
| Citric acid, Anhydrous, Micronized | 1.038 | 1.661 | pH modifier |
| Sodium Lauryl Sulfate | 5.625 | 9.00 | Surfactant |
| Povidone K-25 | 6.25 | 10.00 | Filler/Binder |
| Microcrystalline Cellulose (Avicel PH 112) | 23.483 | 37.573 | Filler/ Binder |
| Anhydrous Lactose | 50.521 | 80.833 | Filler |
| Croscarmellose Sodium | 6.00 | 9.60 | Disintegrant |
| Colloidal Silicon Dioxide (Aerosil 200) | 0.25 | 0.40 | Glidant |
| Magnesium Stearate (Vegetable Extract) | 0.50 | 0.80 | Lubricant |
| **Total (Core Tablet Weight)** | 100 | 160.00 | |
| Opadry II® White 85F18422, HPMC and other inert components | 3.00 | 4.95 | |

### EXAMPLE 7: CCI-779 30 mg Film Coated Tablet Formulation Prepared By Employing Micronized CCI-779

**Table 9: Quantitative Composition of CCI-779 Tablets, 30 mg**

| **Ingredients:** | **Percent Wt/Wt** | **mg** / **tablet** | **Function** |
|---|---|---|---|
| CCI-779, Micronized | 6.25 | 30.000 | Active |
| Butylated Hydroxyanisol, Micronized | 0.022 | 0.105 | Antioxidant |
| Butylated Hydroxytoluene, Micronized | 0.050 | 0.240 | Antioxidant |
| EDTA. Calcium Disodium, Hydrous, Micronized | 0.011 | 0.054 | Chelating agent |
| Citric acid, Anhydrous, Micronized | 1.038 | 4.983 | pH modifier |
| Sodium Lauryl Sulfate | 5.625 | 27.00 | Surfactant |
| Povidone K-25 | 6.25 | 30.000 | Filler/Binder |
| Microcrystalline Cellulose (Avicel PH 112) | 23.483 | 112.718 | Filler/ Binder |
| Anhydrous Lactose | 50.521 | 242.501 | Filler |
| Croscarmellose Sodium | 6.00 | 28.800 | Disintegrant |
| Colloidal Silicon Dioxide (Aerosil 200) | 0.25 | 1.200 | Glidant |
| Magnesium Stearate (Vegetable Extract) | 0.50 | 2.400 | Lubricant |
| **Total (Core Tablet Weight)** | 100 | 480.00 | |
| Opadry II® White 85F18422, HPMC and other inert components | 2.00 | 9.796 | |

## Claims

1. A pharmaceutical composition comprising micronized CCI-779.

2. A pharmaceutical composition according to claim 1, wherein the micronized CCI-779 has a particle size range of 10% are less than or equal to 3 µm, 50% are 10 µm and 90% are less than or equal to 20 µm as determined by Malvern method.

3. A pharmaceutical composition according to claim 1, wherein the micronized CCI has a particle size range of 10% are less than or equal to 2 µm 50% are 5 µm and 90% are less than or equal to 16 µm as determined by Malvern method.

4. A pharmaceutical composition according to any one of claims 1 to 3 which is an immediate release solid dosage form.

5. A composition according to any one of claims 1 to 3 selected from the group consisting of a directly compressible tablet, a capsule, a powder and a suspension.

6. A pharmaceutical composition according to any one of claims 1 to 5, wherein the micronized CCI-779 is present in an amount from 5 % w/w to 10% w/w of the composition.

7. A pharmaceutical composition according to any one of claims 1 to 6, further comprising:
about 5% w/w to about 6.5% w/w surfactant;
about 75 % w/w to about 85% w/w filler/binder;
about 4% w/w to about 6% w/w disintegrant.

8. A pharmaceutical composition according to claim 7, wherein the surfactant is sodium lauryl sulfate.

9. The pharmaceutical composition according to claim 7, wherein the filler/binder is selected from the group consisting of povidone, lactose, and microcrystalline cellulose, and mixtures thereof.

10. The pharmaceutical composition according to claim 7, wherein the disintegrant is croscarmellose sodium.

11. The pharmaceutical composition according to claim 1, further comprising one or more antioxidants, a chelating agent, and/or a pH modifier.

12. The pharmaceutical composition according to claim 11, wherein any one of the one or more antioxidants, a chelating agent and/or pH modifier is micronized.

13. An oral CCI-779 dosing unit comprising micronized CCI-779, a surfactant, a filler/binder, a disintegrant, a glidant, and a lubricant.

14. The oral CCI-779 dosing unit according to claim 13, wherein the micronized CCI-779 has a particle size range of 10% are less than or equal to 2 µm, 50% are 5 µm, and 90% are less than or equal to 16 µm as determined by Malvern method.

15. The oral CCI-779 dosing unit according to claim 13, wherein the micronized CCI-779 is present in an amount from 0.1 % w/w to 10% w/w of the dosing unit, based on total uncoated weight.

16. The oral CCI-779 dosing unit according to claim 13, wherein the surfactant is selected from the group consisting of sodium lauryl sulfate and Poloxamer 188 surfactant.

17. An oral CCI-779 dosing unit according to any one of claims 13 to 16, wherein the filler is selected from the group consisting of microcrystalline cellulose, anhydrous lactose, povidone, and mixtures thereof.

18. An oral CCI-779 dosing unit according to any one of claims 13 to 17, wherein the disintegrant is croscarmellose sodium.

19. An oral CCI-779 dosing unit according to any one of claims 13 to 18, wherein the lubricant is magnesium stearate.

20. An oral CCI-779 dosing unit according to claim 15, comprising:
| | |
|---|---|
| 6 to 7 % w/w | micronized CCI-779; |
| 5 to 7% w/w | surfactant; |
| 50 to 90% w/w | filler; |
| 3 to 8% w/w | disintegrant; |
| less than 1% w/w | glidant; and |
| less than 1% w/w | lubricant. |

21. An oral CCI-779 dosing unit according to claim 20 comprising:
| | |
|---|---|
| CCI-779, Micronized | 6.25% w/w; |
| Sodium Lauryl Sulfate | 5.6% w/w; |
| Povidone | 6.25% w/w; |
| Lactose Anhydrous | 50 % w/w; |
| Microcrystalline Cellulose | 25% w/w; |
| Croscarmellose Sodium | 6% w/w; |
| Glidant | 0.25% w/w; and |
| Magnesium Stearate | 0.25% w/w. |

22. An oral CCI-779 dosing unit according to claim 20 comprising:
| | |
|---|---|
| Micronized CCI-779 | 6 % w/w; |
| Sodium Lauryl Sulfate | 6% w/w; |
| Povidone | 31% w/w; |
| Lactose Anhydrous | 34% w/w; |
| Microcrystalline Cellulose | 16% w/w; |
| Croscarmellose Sodium | 6% w/w ; |
| Glidant | 0.25% w/w; and |
| Magnesium Stearate | 0.5% w/w. |

23. An oral CCI-779 dosing unit according to claim 20 comprising:
| | |
|---|---|
| Micronized CCI-779 | 6% w/w; |
| Butylated Hydroxyanisol | 0.022% w/w; |
| Butylated Hydroxytoluene | 0.05% w/w; |
| EDTA | 0.011% w/w; |
| Citric acid | 0.08% w/w |
| Poloxamer 188 | 6% w/w |
| Lactose Anhydrous | 55% w/w |
| Microcrystalline Cellulose | 28 w/w |
| Croscarmellose Sodium | 4% w/w |
| Glidant | 0.25% w/w; and |
| Magnesium Stearate | 0.5% w/w. |

24. An oral CCI-779 dosing unit according to claim 20 comprising:
| | |
|---|---|
| CCI-779 (Micronized) | 6% w/w; |
| Butylated Hydroxyanisole (Micronized) | 0.022% w/w; |
| Butylated Hydroxytoluene (Micronized) | 0.050% w/w; |
| EDTA Calcium Disodium, Hydrous (Micronized) | 0.011% w/w; |
| Citric Acid Anhydrous (Micronized) | 1% w/w; |
| Sodium Lauryl Sulfate | 6% w/w; |
| Povidone | 6% w/w; |
| Microcrystalline Cellulose | 24% w/w; |
| Anhydrous Lactose | 51% w/w; |
| Croscarmellose Sodium | 6% w/w; |
| Colloidal Silicone Dioxide | 0.25% w/w; and |
| Magnesium Stearate | 0.5% w/w. |

25. An oral CCI-779 dosing unit according to any of claims 13 to 24, wherein said dosing unit further comprises a seal coat.

26. An oral CCI-779 dosing unit according to claim 25, wherein said seal comprises about 2% w/w hydroxypropylmethylcellulose of the coated composition.

27. An oral CCI-779 dosing unit according to any of claims 13 to 26, wherein said dosing unit is selected from the group consisting of a tablet and a capsule.

28. Use of micronized CCI-779 in preparing a medicament.

29. Use according to claim 28, wherein said micronized CCI-779 is directly compressed to form the medicament.

30. Use of micronized CCI-779 in preparing an oral dosing unit according to any of claims 13 to 27.

## Patentansprüche

1. Pharmazeutische Zusammensetzung, die mikronisiertes CCI-779 umfasst.

2. Pharmazeutische Zusammensetzung nach Anspruch 1, wobei das mikronisierte CCI-779 einen Partikelgrößenbereich aufweist, bei dem 10% kleiner als oder gleich 3 µm sind, 50% 10 µm betragen und 90% kleiner als oder gleich 20 µm sind gemäß Bestimmung durch die Malvern-Methode.

3. Pharmazeutische Zusammensetzung nach Anspruch 1, wobei das mikronisierte CCI einen Partikelgrößenbereich aufweist, bei dem 10% kleiner als oder gleich 2 µm sind, 50% 5 µm betragen und 90% kleiner als oder gleich 16 µm sind gemäß Bestimmung durch die Malvern-Methode.

4. Pharmazeutische Zusammensetzung nach Anspruch 1 bis 3, die eine Feststoffdarreichungsform zur unmittelbaren Freisetzung darstellt.

5. Zusammensetzung nach Anspruch 1 bis 3, die aus der aus einer direkt komprimierbaren Tablette, einer Kapsel, einem Pulver und einer Suspension bestehenden Gruppe ausgewählt ist.

6. Pharmazeutische Zusammensetzung nach irgendeinem der Ansprüche 1 bis 5, wobei das mikronisierte CCI-779 in einer Menge von 5 % w/w bis 10% w/w der Zusammensetzung vorliegt.

7. Pharmazeutische Zusammensetzung nach irgendeinem der Ansprüche 1 bis 6, die weiter umfasst:
etwa 5% w/w bis etwa 6,5% w/w einer grenzflächenaktiven Substanz;
etwa 75 % w/w bis etwa 85% w/w eines Füll-/Bindemittels;
etwa 4% w/w bis etwa 6% w/w eines Zerkleinerungsmittels.

8. Pharmazeutische Zusammensetzung nach Anspruch 7, wobei die grenzflächenaktive Substanz Natriumlaurylsulfat ist.

9. Pharmazeutische Zusammensetzung nach Anspruch 7 oder 8, wobei das Füll-/Bindemittel aus der aus Povidon, Lactose und mikrokristalliner Cellulose und Mixturen davon bestehenden Gruppe ausgewählt ist.

10. Pharmazeutische Zusammensetzung nach irgendeinem der Ansprüche 7 bis 9, wobei das Zerkleinerungsmittel Croscarmellose-Natrium ist.

11. Pharmazeutische Zusammensetzung nach irgendeinem der Ansprüche 1 bis 11, die weiter ein Antioxidans oder mehrere Antioxidanzien, ein chelatbildendes Mittel und/oder einen pH-Modifizierer umfasst.

12. Pharmazeutische Zusammensetzung nach Anspruch 11, wobei das Antioxidans oder irgendeines der mehreren Antioxidanzien, des chelatbildenden Mittels und/oder pH-Modifizierers mikronisiert sind.

13. Orale CCI-779-Einheitsdarreichungsform, die mikronisiertes CCI-779, eine grenzflächenaktive Substanz, ein Füll-/Bindemittel, ein Zerkleinerungs-, ein Gleit- und ein Schmiermittel umfasst.

14. Orale CCI-779-Einheitsdarreichungsform nach Anspruch 13, wobei das mikronisierte CCI-779 einen Partikelgrößenbereich aufweist, bei dem 10% kleiner als oder gleich 2 µm sind, 50% 5 µm betragen und 90% kleiner als oder gleich 16 µm sind gemäß Bestimmung durch die Malvern-Methode.

15. Orale CCI-779-Einheitsdarreichungsform nach Anspruch 13, oder 14 wobei das mikronisierte CCI-779 in einer Menge von 0,1 % w/w bis 10% w/w der Einheitsdarreichungsform basierend auf dem unüberzogenen Gesamtgewicht vorliegt.

16. Orale CCI-779-Einheitsdarreichungsform nach irgendeinem der Ansprüche 13 bis 15, wobei die grenzflächenaktive Substanz aus der aus Natriumlaurylsulfat und der grenzflächenaktiven Substanz Poloxamer 188 bestehenden Gruppe ausgewählt ist.

17. Orale CCI-779-Einheitsdarreichungsform nach irgendeinem der Ansprüche 13 bis 16, wobei das Füllmittel aus der aus mikrokristalliner Cellulose, wasserfreier Lactose, Povidon und Mixturen davon bestehenden Gruppe ausgewählt ist.

18. Orale CCI-779-Einheitsdarreichungsform nach irgendeinem der Ansprüche 13 bis 17, wobei das Zerkleinerungsmittel Croscarmellose-Natrium ist.

19. Orale CCI-779-Einheitsdarreichungsform nach irgendeinem der Ansprüche 13 bis 18, wobei das Schmiermittel Magnesiumstearat ist.

20. Orale CCI-779-Einheitsdarreichungsform nach Anspruch 15 umfassend:
6 bis 7 % w/w des mikronisierten CCI-779;
5 bis 7% w/w der grenzflächenaktiven Substanz;
50 bis 90% w/w des Füllmittels;
3 bis 8% w/w des Zerkleinerungsmittels;
weniger als 1% w/w des Gleitmittels und
weniger als 1% w/w des Schmiermittels.

21. Orale CCI-779-Einheitsdarreichungsform nach Anspruch 20 umfassend:
| | |
|---|---|
| Mikronisiertes CCI-779 | 6,25% w/w; |
| Natriumlaurylsulfat | 5,6% w/w; |
| Povidon | 6,25% w/w; |
| Wasserfreie Lactose | 50% w/w; |
| Mikrokristalline Cellulose | 25% w/w; |
| Croscarmellose-Natrium | 6% w/w; |
| Gleitmittel | 0,25% w/w und |
| Magnesiumstearat | 0,25% w/w. |

22. Orale CCI-779-Einheitsdarreichungsform nach Anspruch 20 umfassend:
| | |
|---|---|
| Mikronisiertes CCI-779 | 6% w/w; |
| Natriumlaurylsulfat | 6% w/w; |
| Povidon | 31 % w/w; |
| Wasserfreie Lactose | 34% w/w; |
| Mikrokristalline Cellulose | 16% w/w; |
| Croscarmellose-Natrium | 6% w/w; |
| Gleitmittel | 0,25% w/w und |
| Magnesiumstearat | 0,5% w/w. |

23. Orale CCI-779-Einheitsdarreichungsform nach Anspruch 20 umfassend:
| | |
|---|---|
| Mikronisiertes CCI-779 | 6% w/w; |
| Butyliertes Hydroxyanisol | 0,022% w/w; |
| Butyliertes Hydroxytoluen | 0,05% w/w; |
| EDTA | 0,011 % w/w; |
| Zitronensäure | 0,08% w/w |
| Poloxamer 188 | 6% w/w |
| Wasserfreie Lactose | 55% w/w |
| Mikrokristalline Cellulose | 28 w/w |
| Croscarmellose-Natrium | 4% w/w |
| Gleitmittel | 0,25% w/w und |
| Magnesiumstearat | 0,5% w/w. |

24. Orale CCI-779-Einheitsdarreichungsform nach Anspruch 20 umfassend:
| | | |
|---|---|---|
| CCI-779 (mikronisiert) | | 6% w/w; |
| Butyliertes Hydroxyanisol (mikronisiert) | | 0,022% w/w; |
| Butyliertes Hydroxytoluen (mikronisiert) | | 0,050% w/w; |
| EDTA Kalziumdinatrium, hydriert (mikronisiert) | | 0,011 % w/w; |
| Wasserfreie Zitronensäure (mikronisiert) | | 1 % w/w; |
| | Natriumlaurylsulfat | 6% w/w; |
| | Povidon | 6% w/w; |
| | Mikrokristalline Cellulose | 24% w/w; |
| | Wasserfreie Lactose | 51 % w/w; |
| | Croscarmellose-Natrium | 6% w/w; |
| | Kolloidales Silicondioxid | 0,25% w/w |
| und | | |
| | Magnesiumstearat | 0,5% w/w. |

25. Orale CCI-779-Einheitsdarreichungsform nach irgendeinem der Ansprüche 13 bis 24, wobei die Einheitsdarreichungsform weiter einen Versiegelungsüberzug umfasst.

26. Orale CCI-779-Einheitsdarreichungsform nach Anspruch 25, wobei der Versiegelungsüberzug etwa 2% w/w der Hydroxypropylmethylcellulose in der überzogenen Zusammensetzung enthält.

27. Orale CCI-779-Einheitsdarreichungsform nach irgendeinem der Ansprüche 13 bis 26, wobei die Einheitsdarreichungsform aus der aus einer Tablette und einer Kapsel bestehenden Gruppe ausgewählt ist.

28. Verwendung des mikronisierten CCI-779 bei der Zubereitung eines Medikaments.

29. Verwendung nach Anspruch 28 wobei das mikronisierte CCI-779 direkt komprimiert wird, um das Medikament zu bilden.

30. Verwendung des mikronisierten CCI-779 bei der Zubereitung einer oralen Einheitsdarreichungsform nach irgendeinem der Ansprüche 13 bis 27.

## Revendications

1. Composition pharmaceutique comprenant du CCI-779 micronisé.

2. Composition pharmaceutique selon la revendication 1, dans laquelle le CCI-779 micronisé possède une plage granulométrique dans laquelle 10 % possèdent une granulométrie inférieure ou égale à 3 µm, 50 % possèdent une granulométrie de 10 µm, et 90 % possèdent une granulométrie inférieure ou égale à 20 µm, comme déterminé par le procédé de Malvern.

3. Composition pharmaceutique selon la revendication 1, dans laquelle le CCI micronisé possède une plage granulométrique dans laquelle 10 % possèdent une granulométrie inférieure ou égale à 2 µm, 50 % possèdent une granulométrie de 5 µm, et 90 % possèdent une granulométrie inférieure ou égale à 16 µm, comme déterminé par le procédé de Malvern.

4. Composition pharmaceutique selon l'une quelconque des revendications 1 à 3, qui représente une forme posologique solide à libération immédiate.

5. Composition selon l'une quelconque des revendications 1 à 3, choisi parmi le groupe constitué par un comprimé directement compressible, une capsule, une poudre et une suspension.

6. Composition pharmaceutique selon l'une quelconque des revendications 1 à 5, dans laquelle le CCI-779 micronisé est présent en une quantité de 5 % en poids/poids à 10 % en poids/poids de la composition.

7. Composition pharmaceutique selon l'une quelconque des revendications 1 à 6, comprenant en outre :
un agent tensioactif à concurrence d'environ 5 % en poids/poids à environ 6,5 % en poids/poids ;
une matière de charge/un liant à concurrence d'environ 75 % en poids/poids à environ 85 % en poids/poids ;
un délitant à concurrence d'environ 4 % en poids/poids à environ 6 % en poids/poids.

8. Composition pharmaceutique selon la revendication 7, dans laquelle l'agent tensioactif est le laurylsulfate de sodium.

9. Composition pharmaceutique selon la revendication 7 ou la revendication 8, dans laquelle la matière de charge/le liant est choisi parmi le groupe constitué par la povidone, le lactose et la cellulose microcristalline, ainsi que leurs mélanges.

10. Composition pharmaceutique selon l'une quelconque des revendications 7 à 9, dans laquelle le délitant est le croscarmellose sodium.

11. Composition pharmaceutique selon l'une quelconque des revendications 1 à 11, comprenant en outre un ou plusieurs antioxydants, un agent de chélation et/ou un modificateur du pH.

12. Composition pharmaceutique selon la revendication 11, dans laquelle l'un quelconque desdits un ou plusieurs antioxydants, dudit agent de chélation et/ou dudit modificateur de pH est micronisé.

13. Unité posologique de CCI-779 à administration orale comprenant du CCI-779 micronisé, un agent tensioactif, une matière de charge/un liant, un délitant, un agent de glissement et un lubrifiant.

14. Unité posologique de CCI-779 à administration orale selon la revendication 13, dans laquelle le CCI-779 micronisé possède une plage granulométrique dans laquelle 10 % possèdent une granulométrie inférieure ou égale à 2 µm, 50 % possèdent une granulométrie de 5 µm, et 90 % possèdent une granulométrie inférieure ou égale à 16 µm, comme déterminé par le procédé de Malvern.

15. Unité posologique de CCI-779 à administration orale selon la revendication 13 ou la revendication 14, dans laquelle le CCI-779 micronisé est présent en une quantité de 0,1 % en poids/poids à 10 % en poids/poids de l'unité posologique, basés sur le poids total à l'état non enrobé.

16. Unité posologique de CCI-779 à administration orale selon l'une quelconque des revendications 13 à 15, dans laquelle l'agent tensioactif est choisi parmi le groupe constitué par le laurylsulfate de sodium et l'agent tensioactif poloxamère 188.

17. Unité posologique de CCI-779 à administration orale selon l'une quelconque des revendications 13 à 16, dans laquelle la matière de charge est choisie parmi le groupe constitué par la cellulose microcristalline, du lactose anhydre, la povidone, et leurs mélanges.

18. Unité posologique de CCI-779 à administration orale selon l'une quelconque des revendications 13 à 17, dans laquelle le délitant est la croscarmellose sodium.

19. Unité posologique de CCI-779 à administration orale selon l'une quelconque des revendications 13 à 18, dans laquelle le lubrifiant est le stéarate de magnésium.

20. Unité posologique de CCI-779 à administration orale selon la revendication 15, comprenant :
du CCI-779 micronisé à concurrence de 6 à 7 % en poids/poids ;
un agent tensioactif à concurrence de 5 à 7 % en poids/poids ;
une matière de charge à concurrence de 50 à 90 % en poids/poids ;
un délitant à concurrence de 3 à 8 % en poids/poids ;
un agent de glissement à concurrence de moins de 1 % en poids/poids ; et
un lubrifiant à concurrence de moins de 1 % en poids/poids.

21. Unité posologique de CCI-779 à administration orale selon la revendication 20, comprenant :
du CCI-779 micronisé à concurrence de 6,25 % en poids/poids ;
du laurylsulfate de sodium à concurrence de 5,6 % en poids/poids ;
de la povidone à concurrence de 6,25 % en poids/poids ;
du lactose anhydre à concurrence de 50 % en poids/poids;
de la cellulose microcristalline à concurrence de 25 % en poids/poids ;
du croscarmellose sodium à concurrence de 6 % en poids/poids ;
un agent de glissement à concurrence de 0,25 % en poids/poids ; et
du stéarate de magnésium à concurrence de 0,25 % en poids/poids.

22. Unité posologique de CCI-779 à administration orale selon la revendication 20, comprenant :
du CCI-779 micronisé à concurrence de 6 % en poids/poids ;
du laurylsulfate de sodium à concurrence de 6 % en poids/poids ;
de la povidone à concurrence de 31 % en poids/poids ;
du lactose anhydre à concurrence de 34 % en poids/poids ;
de la cellulose microcristalline à concurrence de 16 % en poids/poids ;
du croscarmellose sodium à concurrence de 6 % en poids/poids ;
un agent de glissement à concurrence de 0,25 % en poids/poids ; et
du stéarate de magnésium à concurrence de 0,5 % en poids/poids.

23. Unité posologique de CCI-779 à administration orale selon la revendication 20, comprenant :
du CCI-779 micronisé à concurrence de 6 % en poids/poids ;
de l'hydroxyanisole butylé à concurrence de 0,022 % en poids/poids ;
de l'hydroxytoluène butylé à concurrence de 0,05 % en poids/poids ;
du EDTA à concurrence de 0,011 % en poids/poids ;
de l'acide citrique à concurrence de 0,08 % en poids/poids ;
du poloxamère 188 à concurrence de 6 % en poids/poids ;
du lactose anhydre à concurrence de 55 % en poids/poids ;
de la cellulose microcristalline à concurrence de 28 % en poids/poids ;
du croscarmellose sodium à concurrence de 4 % en poids/poids ;
un agent de glissement à concurrence de 0,25 % en poids/poids ; et
du stéarate de magnésium à concurrence de 0,5 % en poids/poids.

24. Unité posologique de CCI-779 à administration orale selon la revendication 20, comprenant :
du CCI-779 (micronisé) à concurrence de 6 % en poids/poids ;
de l'hydroxyanisole butylé (micronisé) à concurrence de 0,022 % en poids/poids ;
de l'hydroxytoluène butylé (micronisé) à concurrence de 0,050 % en poids/poids ;
du EDTA calcium disodique, hydraté (micronisé) à concurrence de 0,011 % en poids/poids ;
de l'acide citrique anhydre (micronisé) à concurrence de 1 % en poids/poids ;
du laurylsulfate de sodium à concurrence de 6 % en poids/poids ;
de la povidone à concurrence de 6 % en poids/poids ;
de la cellulose microcristalline à concurrence de 24 % en poids/poids;
du lactose anhydre à concurrence de 51 % en poids/poids ;
du croscarmellose sodium à concurrence de 6 % en poids/poids ;
du dioxyde de silicium colloïdal à concurrence de 0,25 % en poids/poids ; et
du stéarate de magnésium à concurrence de 0,5 % en poids/poids.

25. Unité posologique de CCI-779 à administration orale selon l'une quelconque des revendications 13 à 24, dans laquelle ladite unité posologique comprend en outre un enrobage d'étanchéité.

26. Unité posologique de CCI-779 à administration orale selon la revendication 25, ledit enrobage d'étanchéité comprend de l'hydroxypropylméthylcellulose à concurrence d'environ 2 % en poids/poids de la composition enrobée.

27. Unité posologique de CCI-779 à administration orale selon l'une quelconque des revendications 13 à 26, dans laquelle ladite unité posologique est choisie parmi le groupe constitué par un comprimé et une capsule.

28. Utilisation de CCI-779 micronisé pour préparer un médicament.

29. Utilisation selon la revendication 28, dans laquelle ledit CCI-779 micronisé est soumis à une compression directe pour obtenir le médicament.

30. Utilisation de CCI-779 micronisé pour préparer une unité posologique à administration orale conformément à l'une quelconque des revendications 13 à 27.
